# EUROPEAN PATENT APPLICATION

(11) **EP 2 875 774 A1**
(43) Date of publication of application: **27.05.2015**
(21) Application number: 14189786.8
(22) Date of filing: 21.10.2014
(51) Int. Cl.: A61B 1/015, A61B 1/12, A61M 1/00, A61M 3/02

(54) **Endoscopic Apparatus including a Re-Circulation Device and Filter and Method of Supplying an Irrigation Fluid during an Endoscopic Procedure**

(30) Priority: 21.11.2013 US 201361906986 P
(71) Applicant: Cook Medical Technologies LLC, Bloomington, IN 47404 (US)
(72) Inventor: Ryan, Walter N., Greenville, NC North Carolina 27858 (US)
(74) Representative: Garratt, Peter Douglas

(57) **Abstract**

Endoscopic apparatus includes an endoscopic device having a fluid delivery port and a fluid extraction port, and a filter device. The fluid extraction port connects to the filter input port and the filter output port connects to the fluid delivery port forming a fluid circulation path from the fluid extraction port through the filter device to the fluid delivery port. The apparatus also includes a fluid pump positioned in the fluid circulation path. A method of supplying an irrigation fluid during an endoscopic procedure includes delivering the irrigation fluid to the distal end of the endoscopic device, aspirating the irrigation fluid from a region around the distal end, filtering the irrigation fluid and returning the filtered irrigation fluid to the distal end of the endoscopic device.

## Description

### RELATED APPLICATIONS

The present patent application claims the benefit of the filing date under 35 U.S.C. §119(e) of U.S. Provisional Patent Application Serial No. 61/906,986, filed November 21, 2013.

### TECHNICAL FIELD

The present invention relates generally to the field of medical devices, and more particularly, to an endoscopic apparatus having an irrigation re-circulation device and filter. Methods of using such an endoscopic apparatus are also disclosed.

### BACKGROUND

Endoscopy provides a method of examining the inside of a body cavity for medical reasons using an endoscope. For example, many diseases of the urinary tract require endoscopic examination or surgery. These include kidney stones, ureteropelvic junction obstruction, ureteral strictures, tumors in the kidney collecting system or ureter and relief of blockage in the kidney.

Such endoscopic techniques sometimes involve the delivery of an irrigation fluid to the distal end of an endoscope and the aspiration of the fluid from the body cavity. For example, such a fluid can be utilized to remove particulate matter or fluid from the region under endoscopic investigation. For example, blood, mucus or other matter may be removed to improve the endoscopist's visualization and therapeutic capabilities.

Typically, an irrigation fluid is delivered from an irrigation bag to the distal end of the endoscope through a fluid delivery channel. For example, this channel may be positioned within the endoscope body.
Alternatively, the fluid delivery channel may be contained within a separate device. The irrigation fluid is then aspirated from the region around the distal end of the endoscope through a separate fluid removal channel. Again, this channel may be positioned within the endoscope body or may be a component of a separate device. The aspirated fluid is then collected for disposal.

### SUMMARY

One aspect of the present invention provides an endoscopic apparatus including an endoscopic device having a fluid delivery port and a fluid extraction port, a filter device comprising a housing having a filter input port and a filter output port and a filter positioned within the housing. The fluid extraction port connects to the filter input port and the filter output port connects to the fluid delivery port forming a fluid circulation path from the fluid extraction port through the filter device to the fluid delivery port. The apparatus also includes a fluid pump positioned in the fluid circulation path. The endoscopic apparatus can also include a fluid reservoir in fluid communication with the fluid delivery port.

In certain embodiments, the filter device includes a membrane filter, for example, a hollow fiber filter. The filter can include a membrane with pores having a maximum size of between 0.1 microns and 10 microns. The filter device can also include a pre-filter positioned within the filter housing between the filter input port and the filter.

In one embodiment, the endoscopic device includes an elongated member having a fluid delivery port and a fluid extraction port positioned near the proximal end. A fluid delivery channel is contained within the elongated member and extends from the fluid delivery port to the distal end of the elongated member. A fluid extraction channel is also contained within the endoscope member extends from the distal end to the fluid extraction port.

In another embodiment, the endoscopic device includes a fluid delivery device and a fluid extraction device. In one embodiment, the fluid delivery device includes a first elongated member having the fluid delivery port positioned near the proximal end and a fluid delivery channel contained within and extending from the fluid delivery port to the distal end of the first elongated member. The fluid extraction device includes a second elongated member having a fluid extraction port positioned near the proximal end and a fluid extraction channel contained within and extending from the distal end to the fluid extraction port.

Another aspect of the present invention provides a method of supplying an irrigation fluid during an endoscopic examination including delivering the irrigation fluid from a fluid delivery port of an endoscopic device to a distal end of the endoscopic device and aspirating the irrigation fluid from a region around the distal end. The method also includes filtering the irrigation fluid in a filter device and returning the filtered irrigation fluid through the fluid delivery port to the distal end of the endoscopic device.

In one embodiment, the filtering removes discoloration and/or particulate matter from the irrigation fluid.

### BRIEF DESCRIPTION OF THE DRAWINGS

FIG. 1 is a schematic illustrating the components of one embodiment of an endoscopic apparatus in accordance with the present invention.
FIG. 2 is a schematic illustrating the components of another embodiment of an endoscopic apparatus in accordance with the present invention.
FIG. 3 is a schematic illustrating one embodiment of an endoscopic device and sheath for use in the endoscopic apparatus in accordance with the present invention.
FIG. 4 is a schematic illustrating an embodiment of a multi-lumen sheath for use in the endoscopic apparatus in accordance with the present invention.
FIG. 5 is a schematic illustrating the components of yet another embodiment of an endoscopic apparatus in accordance with the present invention.

### DETAILED DESCRIPTION OF THE PREFERRED EMBODIMENTS DEFINITIONS

Unless otherwise defined, all technical and scientific terms used herein have the same meaning as commonly understood by one of ordinary skill in the art to which this invention pertains. In case of conflict, the present document, including definitions, will control. Preferred devices, methods and materials are described below, although devices, methods and materials similar or equivalent to those described herein can be used in the practice of the present invention. All publications, patent applications, patents and other references mentioned herein are incorporated by reference in their entirety.

The uses of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example") provided herein, is intended merely to better illuminate the invention and does not pose a limitation on the scope of the invention unless otherwise claimed. No language in the specification should be construed as indicating any non-claimed element as essential to the practice of the invention.

As used herein the terms "comprise(s)," "include(s)," "having," "has," "can," "contain(s)," and variants thereof, are intended to be openended transitional phrases, terms, or words that do not preclude the possibility of additional acts or structures. The present invention also contemplates other embodiments "comprising," "consisting of" and "consisting essentially of," the embodiments or elements presented herein, whether explicitly set forth or not.

As used in the specification, the terms "proximal" and "distal" should be understood as being in the terms of a physician using the endoscopic device. The term distal means the portion of the device which is farthest from the physician and the term proximal means the portion of the device which is nearest to the physician. Typically, the distal portion of the device will be inserted into the body of the patient undergoing an endoscopic procedure while the proximal portion of the device will remain outside the body of the patient.

As used herein, the term "body cavity" means any body passage or lumen, including but not limited to vascular coronary or peripheral vessels, esophageal, intestinal, biliary, bronchial, reproductive, urethral and ureteral passages. The term also encompasses a body passage formed during a surgical procedure.

For the purpose of promoting an understanding of the principles of the invention, reference will now be made to embodiments, some of which are illustrated in the drawings, and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates.

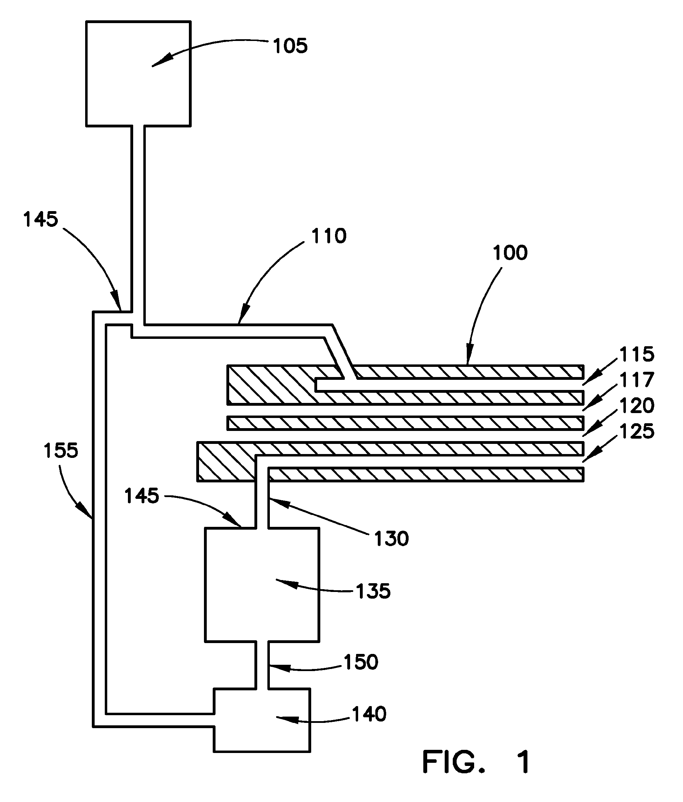

Referring now to Figure 1, one embodiment of the present invention provides an endoscopic apparatus including an endoscopic device 100, a filter device 135 and a fluid pump 140. Fluid delivery port 110 is positioned near the proximal end of endoscopic device 100 and is connected to a proximal region of fluid delivery channel 115. Fluid delivery channel 115 extends distally from fluid delivery port 110 to the distal end of endoscopic device 100. Fluid extraction port 130 is also positioned near the proximal end of endoscopic device 100 and connects to a proximal region of fluid extraction channel 125.

In certain embodiments, endoscopic device 100 also includes one or more additional channels. For example, endoscopic device 100 can include visualization channel 117 containing a fiber optic or a similar optical observation system, allowing the operator to view the region around the distal end of endoscopic device 100 when the distal region of the device is placed within a body cavity of a subject. Endoscopic device 100 can also include instrument channel 120 allowing the insertion of medical instruments or manipulators. For example, instrument channel 120 can allow for the insertion of an instrument used to obtain a tissue sample for biopsy or to destroy small tumors or gallstones.

Fluid delivery port 110 is connected to fluid reservoir 105. Fluid extraction port 130 connects to filter input port 145 of filter device 135. Filter output port 150 of filter device 135 connects to fluid delivery port 110 via fluid return path 155, forming a fluid circulation path from the fluid extraction port 130 through the filter device 135 to the fluid delivery port 110. Fluid pump 140 is shown positioned in fluid return path 155. In other embodiments, fluid pump 140 is positioned between fluid extraction port 130 and filter input port 145. Fluid pump 140 can be a vacuum pump or any other pump type that is suitable to re-circulate the irrigation fluid through filter device 135 and return the irrigation fluid to fluid delivery port 110.

Filter device 135 can include any filter medium suitable for the removal of particulate matter. For example, filter device 135 can include a membrane filter having a maximum pore size of between 0.1 microns and 10 microns, or between 0.1 and 1 microns, or between 0.1 and 0.5 microns or between 0.1 and 0.2 microns. In certain embodiments, the filter is a hollow fiber filter. Such a filter can include long and straight hollow fibers formed from, for example, polyethersulfone, bundled into the filter housing.

Filter device 135 can also include a pre-filter positioned between filter input port 145 and the filter medium. In such an embodiment, larger particulate matter is removed by the pre-filter before the irrigation fluid is further filtered to remove smaller particles and/or discoloration.

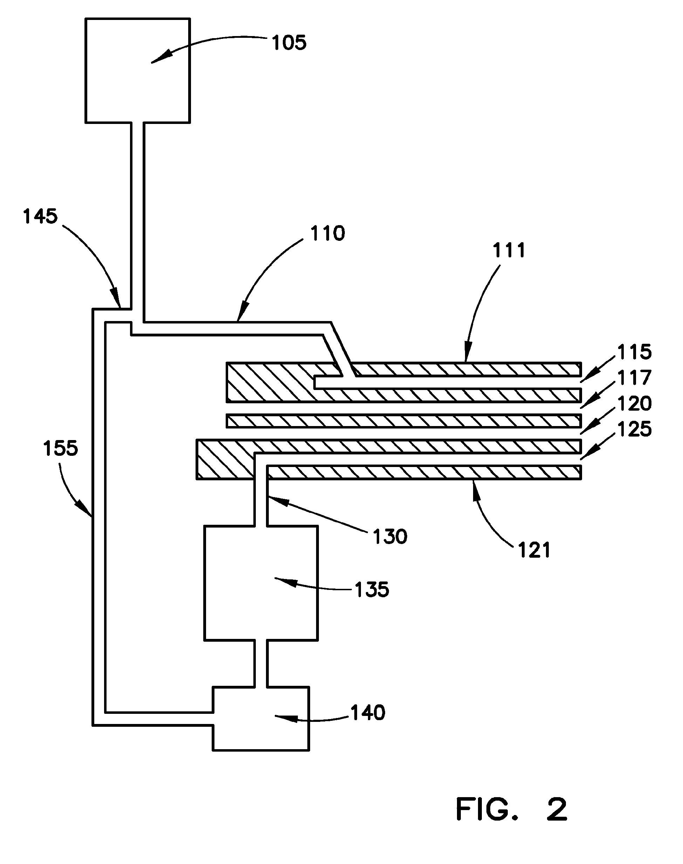

Referring now to Figure 2, this figure illustrates another embodiment of an endoscopic apparatus of the present invention. Here, the endoscopic device includes an irrigation fluid delivery device 111 and a separate fluid extraction device 121. The endoscopic apparatus also includes filter device 135 and fluid pump 140.

Fluid delivery port 110 of fluid delivery device 111 connects to fluid reservoir 105 and allows for the delivery of irrigation fluid from fluid reservoir 105 to the distal end of the device via fluid delivery channel 115. On some embodiments, fluid delivery device 111 also includes one of more additional channels. For example, as is illustrated in Figure 2, the device can include visualization channel 117 containing a fiber optic or a similar optical observation system. Device 111 can also include instrument channel 120 allowing the insertion of medical instruments or manipulators. In other embodiments, fluid delivery device 111 does not include such additional channels. In these embodiments, the endoscopic apparatus includes an additional endoscopic device including a visualization and/or instrument channel.

Fluid extraction device 121 connects to filter device 135 via fluid extraction port 130. Irrigation fluid is aspirated from the distal end of device 121 through fluid aspiration channel 125 to fluid extraction port 130 and through filter device 135. After filtration, the filtered irrigation fluid is returned to fluid delivery port 110 of fluid delivery device 111 via fluid return path 155.

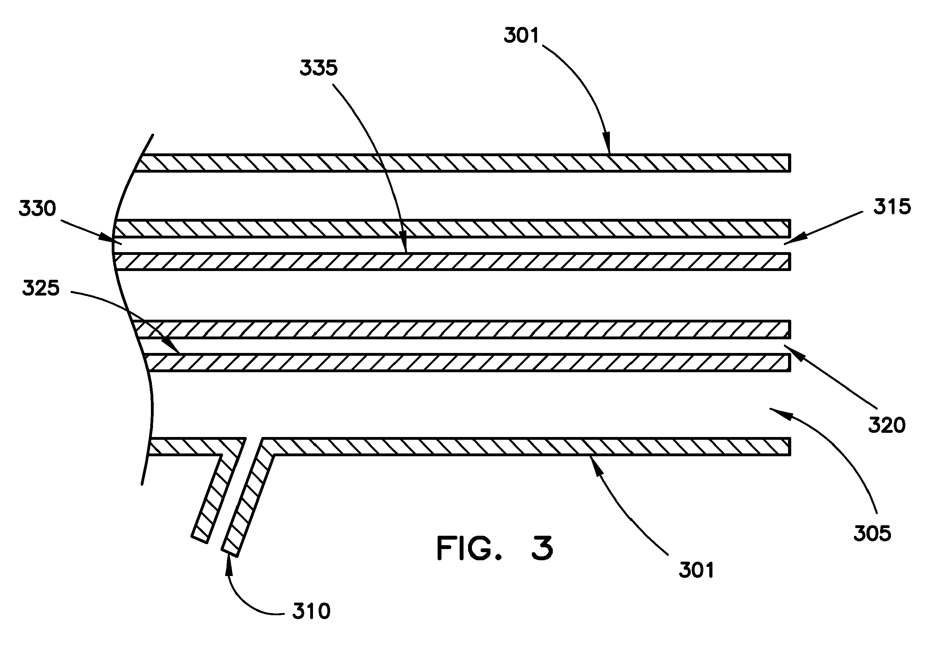

Referring now to Figure 3, this figure illustrates one embodiment of a portion of an endoscopic device and sheath for use in the endoscopic apparatus of the present invention. This device includes a sheath 301 having fluid delivery device 315 positioned within lumen 305. Endoscope 320 having lumen 325 for a visualization and/or an instrument is also positioned within lumen 305. Endoscope 320 can include one or more additional lumens (not shown.) For example, the endoscope can include separate lumens for visualization and instrument delivery

Delivery device 315 includes fluid delivery port 330 positioned towards the proximal end of the device and lumen 335 extending from fluid delivery port 330 to the distal end of the device. Fluid extraction port 310 is positioned towards the proximal end of sheath 301.

Fluid delivery port 330 connects to fluid reservoir 105 (shown, for example, in FIG. 1), allowing for the delivery of irrigation fluid to the distal end of fluid delivery device 315. Fluid extraction port 310 connects to filter device 135, allowing for the aspiration of the irrigation fluid from lumen 305 of sheath 310, filtration of the irrigation fluid and return of the fluid to fluid delivery port 330 (again shown, for example, in FIG. 1.)

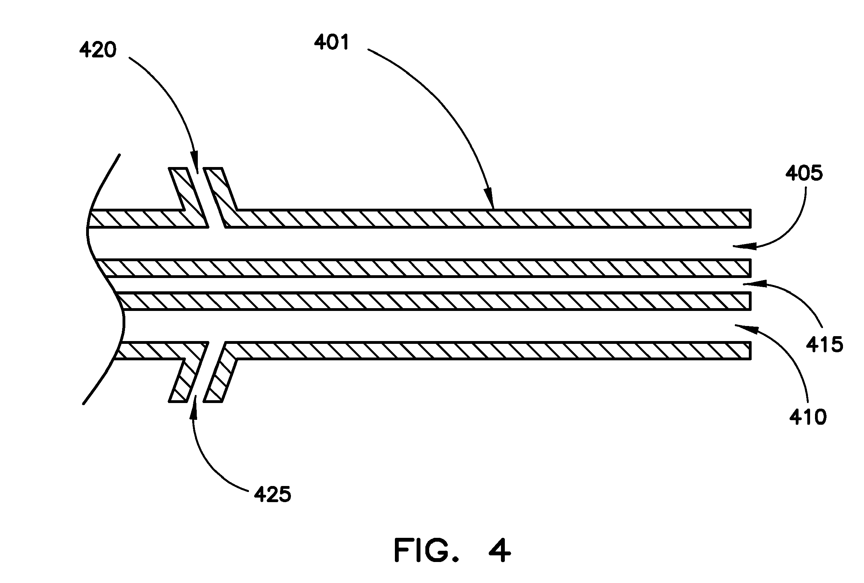

Referring now to Figure 4, this figure illustrates another embodiment of a portion of an sheath suitable for use in the endoscopic apparatus of the present invention. Here, sheath 401 is a multi-lumen sheath including, at least, fluid delivery lumen 405, fluid extraction lumen 410 and lumen 415. An endoscope may be positioned within lumen 415. Fluid delivery port 420 is positioned towards the distal end of sheath 401 and connects to fluid delivery lumen 405, allowing for the delivery of irrigation fluid from fluid reservoir 105. Fluid extraction port 425 is positioned towards the distal end of fluid extraction lumen 410 and allows for the aspiration of irrigation fluid and the delivery of the aspiration fluid to filter device 135 and the return of the irrigation fluid to fluid delivery port 420.

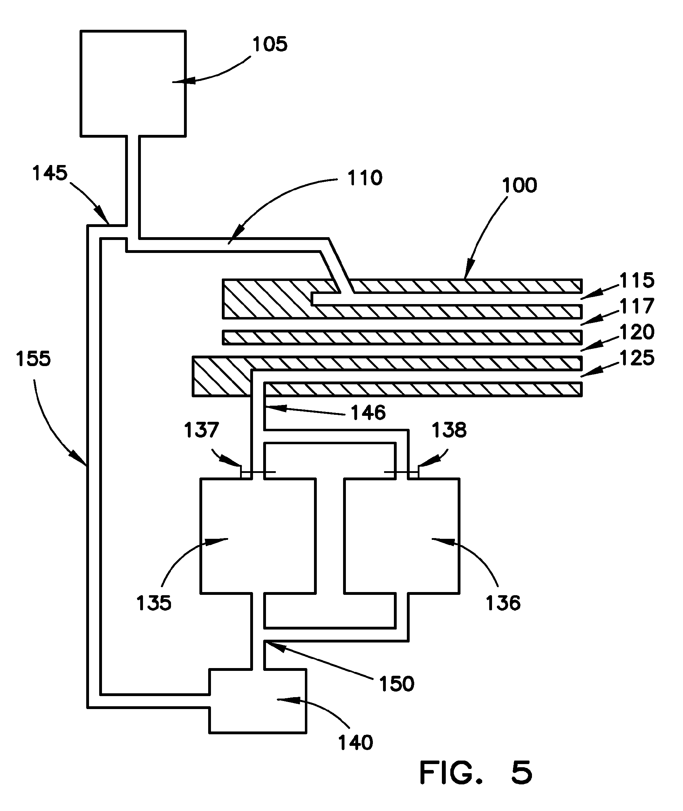

Referring now to FIG. 5, this figure illustrates another embodiment of the endoscopic apparatus in accordance with the present invention. Here, filter input port 146 connects to filter units 135 and 138, which are positioned in parallel. Valves 137 and 138 allow for the direction of aspirated irrigation fluid to either filter unit 135 or filter unit 138, or to both filter units. Configurations including 3, 4, 5, 6 or more filter units are also within the scope of the present embodiments. In certain embodiments, valves 137 and 138 are replaced by a multi-position valve, allowing for the delivery of irrigation fluid to either filter unit. In certain embodiments, the input and output ports of the filter units include connectors allowing for the replacement of one or more of the filter units during an endoscopic procedure.

Another aspect of the present invention provides a method for supplying an irrigation fluid during an endoscopic procedure performed using one of the endoscopic devices disclosed above. Certain endoscopic procedures, particularly those having a long duration, can require the supply of large amounts of irrigation fluid. This can require the replacement of multiple bags containing the irrigation fluid. In one embodiment, the apparatus disclosed herein reduces the need for multiple bags of irrigation fluid by providing a means of filtering and re-using irrigation fluid aspirated from the distal region of the endoscopic device. Such a system can provide cost savings, eliminate waste and reduce labor requirements. Re-use of the irrigation also reduces the need to equilibrate fresh irrigation fluid to body temperature to reduce or prevent fogging of any observation system included in the endoscopic device.

The endoscopic procedure can be a procedure including the introduction of the distal portion of one of the endoscopic devices or apparatus disclosed above onto a body cavity of a subject. For example, the body cavity can be an esophageal, an intestinal, a biliary, a bronchial, a reproductive, an urethral or an ureteral passage. In certain embodiments, an access sheath facilitates the passage of endoscopic device into the body cavity.

Referring, for example, to Figure 1, the endoscopic apparatus provides for the re-circulation of irrigation fluid through endoscopic device 100. Irrigation fluid is delivered from fluid reservoir 105 to fluid delivery port 110 and fluid delivery channel 115 to the distal end of endoscopic device 100. The irrigation fluid, along with fluid or particulate matter present in the region around the distal end is then aspirated, by fluid pump 140, through the distal end of fluid aspiration channel 125 to fluid extraction port 130 and through filter device 135. After filtration, the filtered irrigation fluid is returned to fluid delivery port 110 of endoscopic device 100 via fluid return path 155. In some embodiments, fluid return path 155 returns the filtered irrigation fluid to fluid reservoir 105. In other embodiments, the filtered irrigation fluid is returned to fluid delivery port 110 via return connection 145.

In one embodiment, the irrigation fluid is an aqueous irrigation fluid. For example, the irrigation fluid can be water or saline. In certain embodiments, particles obscuring operator vision, for example, surgical debris and/or blood components, are suspended in the irrigation fluid and delivered to the filter device.

Although the invention has been described and illustrated with reference to specific illustrative embodiments thereof, it is not intended that the invention be limited to those illustrative embodiments. Those skilled in the art will recognize that variations and modifications can be made without departing from the true scope and spirit of the invention as defined by the claims that follow. It is therefore intended to include within the invention all such variations and modifications as fall within the scope of the appended claims and equivalents thereof.

## Claims

1. An endoscopic apparatus comprising:
an endoscopic device having a fluid delivery port and a fluid extraction port;
a filter device comprising a housing having a filter input port and a filter output port and a filter positioned within the housing, wherein the fluid extraction port connects to the filter input port and wherein the filter output port connects to the fluid delivery port forming a fluid circulation path from the fluid extraction port through the filter device to the fluid delivery port; and
a fluid pump positioned in the fluid circulation path.

2. The endoscopic apparatus of claim 1, further comprising a fluid reservoir, wherein the fluid reservoir is in fluid communication with the fluid delivery port.

3. The endoscopic apparatus of claim 1, wherein the endoscopic device comprises:
an elongated member having an proximal end and a distal end,
wherein the fluid delivery port and the fluid extraction port are positioned near the proximal end;
a fluid delivery channel positioned within the elongated member and extending from the fluid delivery port to the distal end;
a fluid extraction channel positioned within the elongated member and extending from the distal end to the fluid extraction port.

4. The endoscopic apparatus of claim 1, wherein the endoscopic device comprises:
(a) a fluid delivery device comprising:
a first elongated member having a proximal end and a distal end,
wherein the fluid delivery port is positioned near the proximal end of the first elongated member, and
a fluid delivery channel positioned within the first elongated member, and extending from the fluid delivery port to the distal end of the first elongated member, and
(b) a fluid extraction device comprising:
a second elongated member having a proximal end and a distal end,
wherein the fluid extraction port is positioned near the proximal end of the second elongated member, and
a fluid extraction channel positioned within the second elongated member and extending from the distal end of the second elongated member to the fluid extraction port.

5. The endoscopic apparatus of any one of the preceding claims, wherein the filter is a membrane filter and preferably wherein the membrane filter is a hollow fiber filter.

6. The endoscopic apparatus of claim 5, wherein the hollow fiber filter comprises a membrane comprising pores having a maximum size of between 0.1 microns and 10 microns.

7. The endoscopic apparatus of claim 5, wherein the filter device further comprises a pre-filter positioned within the housing between the filter input port and the hollow fiber filter.

8. The endoscopic apparatus of any one of the preceding claims, further comprising an access sheath.

9. The endoscopic apparatus of any one of the preceding claims, wherein the fluid pump is a vacuum pump.

10. A method of supplying an irrigation fluid during an endoscopic examination comprising:
delivering the irrigation fluid from a fluid delivery port of an endoscopic device to a distal end of the endoscopic device,
aspirating the irrigation fluid from a region around the distal end; delivering the aspirated irrigation fluid to a filter device;
filtering the aspirated irrigation fluid in the filter device to provide a filtered irrigation fluid; and
delivering the filtered irrigation fluid through the fluid delivery port to the distal end of the endoscopic device.

11. The method of claim 10, wherein the filtering comprises passing the aspirated irrigation fluid through a hollow fiber filter.

12. The method of claim 10, wherein the filtering comprises removing discoloration or particulate matter from the irrigation fluid.

13. The method of any one of claim 10 to claim 12, wherein the irrigation fluid is an aqueous irrigation fluid, preferably selected from the group consisting of water and saline.

14. The method of any one of claim 10 to claim 13, wherein the endoscopic device comprises
an elongated member having an proximal end and a distal end and having the fluid delivery port and a fluid extraction port are positioned near the proximal end;
a fluid delivery channel positioned within the elongated member and extending from the fluid delivery port to the distal end;
a fluid extraction channel positioned within the elongated member and extending from the distal end to the fluid extraction port;
a filter device comprising a housing having a filter input port and a filter output port and a filter positioned within the housing, wherein the fluid extraction port connects to the filter input port and wherein the filter output port connects to the fluid delivery port forming a fluid circulation path from the fluid extraction port through the filter device to the fluid delivery port; and
a fluid pump positioned in the fluid circulation path.

15. The method of any one of claim 10 to claim 14, wherein particles obscuring operator vision are suspended in the irrigation fluid and delivered to the filter device.
